(19) Europäisches Patentamt
European Patent Office
Office européen des brevets

(11) EP 1 785 422 B1

(12) # EUROPÄISCHE PATENTSCHRIFT

(45) Veröffentlichungstag und Bekanntmachung des Hinweises auf die Patenterteilung:
**16.11.2016 Patentblatt 2016/46**

(51) Int Cl.:
***C07D 451/10*** *(2006.01)* ***A61P 11/00*** *(2006.01)*
***A61K 31/40*** *(2006.01)*

(21) Anmeldenummer: **07102206.5**

(22) Anmeldetag: **10.03.2003**

(54) **Kristallines Mikronisat des Tiotropiumbromids**

Micronised crystalline tiotropium bromide

Micronisat crystallin de bromure de tiotropium

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**LT LV**

(30) Priorität: **20.03.2002 DE 10212264**

(43) Veröffentlichungstag der Anmeldung:
**16.05.2007 Patentblatt 2007/20**

(60) Teilanmeldung:
**16159836.2 / 3 053 921**

(62) Dokumentnummer(n) der früheren Anmeldung(en) nach Art. 76 EPÜ:
**03708206.2 / 1 487 832**

(73) Patentinhaber: **Boehringer Ingelheim Pharma GmbH & Co. KG**
**55216 Ingelheim (DE)**

(72) Erfinder:
- **Trunk, Michael**
  **55218, Ingelheim (DE)**
- **Walz, Michael**
  **55411, Bingen (DE)**
- **Bender, Helmut**
  **65193, Wiesbaden (DE)**
- **Graebner, Hagen**
  **55218, Ingelheim (DE)**
- **Schindler, Konrad**
  **55218, Ingelheim (DE)**

(74) Vertreter: **Hoffmann Eitle**
**Patent- und Rechtsanwälte PartmbB**
**Arabellastraße 30**
**81925 München (DE)**

(56) Entgegenhaltungen:
**EP-A- 0 418 716** **WO-A1-00/47200**
**FR-A- 2 779 347**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 785 422 B1

## Beschreibung

Die Erfindung betrifft ein kristallines Mikronisat von (1α,2β,4β,5α,7β)-7-[(hydroxydi-2-thienylacetyl)oxy]-9,9-dimethyl-3-oxa-9-azoniatricyclo[3.3.1.0$^{2,4}$]nonane-bromid, sowie dessen Verwendung zur Herstellung eines Arzneimittels, insbesondere zur Herstellung eines Arzneimittels mit anticholinerger Wirkung.

Hintergrund der Erfindung

Die Verbindung (1α,2β,4β,5α,7β)-7-[(hydroxydi-2-thienylacetyl)oxy]-9,9-dimethyl-3-oxa-9-azoniatricyclo[3.3.1.0$^{2,4}$]nonane-bromid, ist aus der Europäischen
Patentanmeldung EP 418 716 A1 bekannt und weist die folgende chemische Struktur auf:

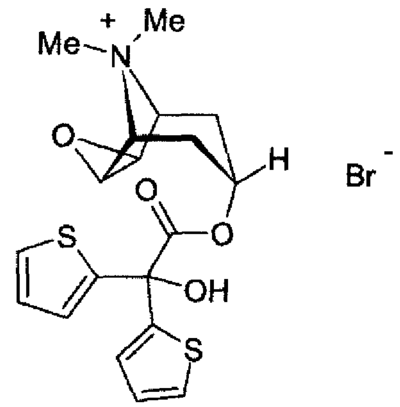

(I)

Die Verbindung besitzt wertvolle pharmakologische Eigenschaften und ist unter dem Namen Tiotropiumbromid (BA679) bekannt. Tiotropiumbromid stellt ein hochwirksames Anticholinergikum dar und kann deshalb bei der Therapie von Asthma oder COPD (chronic obstructive pulmonary disease = chronisch obstruktive Lungenerkrankung) einen therapeutischen Nutzen entfalten.

Die Applikation von Tiotropiumbromid erfolgt vorzugsweise auf inhalativem Wege. Hierbei können geeignete Inhalationspulver, die in geeignete Kapseln (Inhaletten) abgefüllt mittels entsprechender Pulverinhalatoren appliziert werden, zum Einsatz kommen. Alternativ dazu kann eine inhalative Anwendung auch durch Applikation geeigneter Inhalationsaerosole erfolgen. Hierzu zählen auch pulverförmige Inhalationsaerosole, die beispielsweise HFA134a, HFA227 oder deren Gemisch als Treibgas enthalten.

Im Hinblick auf die inhalative Applikation von Tiotropiumbromid, ist es erforderlich, den Wirkstoff in feinteiliger (bzw. mikronisierter) Form bereitzustellen. Vorzugsweise weist der Wirkstoff dabei eine mittlere Teilchengröße von 0,5 bis 10μm, bevorzugt von 1 bis 6μm, besonders bevorzugt von 1,5 bis 5μm auf.
Die vorstehend genannten Teilchengrößen werden in der Regel durch Mahlen (sogenanntes Mikronisieren) des Wirkstoffs erzielt. Die Abfüllung von zuvor mikronsiertem Tiotropiumbromid als Teil einer Inhalationspulverformulierung in entsprechende Gelatinekapseln zur Verwendung in einem Pulverinhalator wird z.B. in der internationalen Patentanmeldung WO 00/47200 beschrieben (vgl. Beispiel 93).
Da als Begleiterscheinung des Mikronisierens trotz der beim Verfahrensablauf erforderlichen harten Bedingungen ein Abbau des Arzneimittelwirkstoffs weitestgehend vermieden werden muß, stellt eine hohe Stabilität des Wirkstoffs gegenüber dem Mahlvorgang eine unabdingbare Notwendigkeit dar. Dabei muß berücksichtigt werden, daß im Zuge des Mahlvorgangs unter Umständen Veränderungen der Feststoffeigenschaften des Wirkstoffs auftreten können, die einen Einfluß auf die pharmakologischen Eigenschaften der inhalativ zu applizierenden Arzneimittelform haben können.

Verfahren zur Mikronisierung von Arzneimittelwirkstoffen sind als solche im Stand der Technik bekannt, z.B. aus FR 2 779 347. Es ist nun Aufgabe der vorliegenden Erfindung, mikronisiertes Tiotropiumbromid in einer Form zugänglich zu machen, die den hohen, an einen inhalativ applizierten Wirkstoff zu richtenden Anforderungen genügt und dabei den spezifischen Eigenschaften des Tiotropiumbromids Rechnung trägt.

Detaillierte Beschreibung der Erfindung

Es wurde gefunden, daß Tiotropiumbromid je nach Wahl der Bedingungen, die bei der Reinigung des nach der technischen Herstellung erhaltenen Rohprodukts angewendet werden können, in unterschiedlichen kristallinen Modifikationen, sogenannten Polymorphen, anfällt.

Es wurde ferner gefunden, daß diese unterschiedlichen Modifikationen maßgeblich durch Wahl der zur Kristallisation eingesetzten Lösemittel sowie durch Wahl der beim Kristallisationsprozess gewählten Verfahrensbedingungen gezielt erhalten werden können.

Für den Zweck der vorliegenden Erfindung, Tiotropiumbromid in für die Inhalation geeigneter, mikronisierter Form bereitzustellen, erwies sich das kristalline Monohydrat des Tiotropiumbromids, welches durch die Wahl spezifischer Reaktionsbedingungen in kristalliner Form erhalten werden kann, besonders geeignet.

Zur Herstellung dieses kristallinen Monohydrats ist es erforderlich, Tiotropiumbromid, welches beispielsweise nach der in der EP 418 716 A1 offenbarten Herstellungsvorschrift erhalten worden ist, in Wasser aufzunehmen, zu Erwärmen, eine Reinigung mit Aktivkohle durchzuführen und nach Abtrennen der Aktivkohle unter langsamem Abkühlen das Tiotropiumbromid-monohydrat langsam zu kristallisieren. Bevorzugt wird wie nachfolgend beschrieben vorgegangen. In einem geeignet dimensionierten Reaktionsgefäß wird das Lösemittel mit Tiotropiumbromid, welches beispielsweise nach der in der EP 418 716 A1 offenbarten Herstellungsvorschrift erhalten worden ist, gemischt.

Pro Mol eingesetztes Tiotropiumbromid werden 0,4 bis 1,5 kg, bevorzugt 0,6 bis 1 kg, besonders bevorzugt ca. 0,8 kg Wasser als Lösemittel verwendet.

Die erhaltene Mischung wird unter Rühren erwärmt, vorzugsweise auf mehr als 50°C, besonders bevorzugt auf mehr als 60°C. Die maximal wählbare Temperatur bestimmt sich durch den Siedepunkt des verwendeten Lösemittels Wasser. Vorzugsweise wird die Mischung auf einen Bereich von 80-90°C erhitzt.

In diese Lösung wird Aktivkohle, trocken oder wasserfeucht, eingebracht. Bevorzugt werden pro Mol eingesetztes Tiotropiumbromid 10 bis 50 g, besonders bevorzugt 15 bis 35 g, höchst bevorzugt etwa 25 g Aktivkohle eingesetzt. Gegebenenfalls wird die Aktivkohle vor Einbringen in die Tiotropiumbromid-haltige Lösung in Wasser aufgeschlämmt. Pro Mol eingesetztes Tiotropiumbromid werden zum Aufschlämmen der Aktivkohle 70 bis 200 g, bevorzugt 100 bis 160 g, besonders bevorzugt ca. 135 g Wasser verwendet. Wird die Aktivkohle vor Einbringen in die Tiotropiumbromid-haltige Lösung zuvor in Wasser aufgeschlämmt, empfiehlt es sich, mit der gleichen Menge Wasser nachzuspülen.

Bei konstanter Temperatur wird nach erfolgter Aktivkohlezugabe zwischen 5 bis 60 Minuten, bevorzugt zwischen 10 und 30 Minuten, besonders bevorzugt etwa 15 Minuten weitergerührt und die erhaltene Mischung filtriert, um die Aktivkohle zu entfernen. Der Filter wird anschließend mit Wasser nachgespült. Hierfür werden pro Mol eingesetztes Tiotropiumbromid 140 bis 400 g, bevorzugt 200 bis 320 g, höchst bevorzugt ca. 270 g Wasser verwendet.

Das Filtrat wird anschließend langsam abgekühlt, vorzugsweise auf eine Temperatur von 20-25°C. Die Abkühlung wird vorzugsweise mit einer Abklühlrate von 1 bis 10°C pro 10 bis 30 Minuten, bevorzugt von 2 bis 8°C pro 10 bis 30 Minuten, besonders bevorzugt von 3 bis 5°C pro 10 bis 20 Minuten, höchst bevorzugt von 3 bis 5°C pro ca. 20 Minuten durchgeführt. Gegebenenfalls kann sich nach dem Abkühlen auf 20 bis 25°C eine weitere Abkühlung auf unter 20°C, besonders bevorzugt auf 10 bis 15°C anschließen.

Nach erfolgter Abkühlung wird zwischen 20 Minuten und 3 Stunden, vorzugsweise zwischen 40 Minuten und 2 Stunden, besonders bevorzugt etwa eine Stunde zur Vervollständigung der Kristallisation nachgerührt.

Die entstandenen Kristalle werden abschließend durch Filtrieren oder Absaugen des Lösemittels isoliert. Sollte es erforderlich sein, die erhaltenen Kristalle einem weiteren Waschschritt zu unterwerfen, empfiehlt es sich als Waschlösemittel Wasser oder Aceton zu verwenden. Pro Mol eingesetztes Tiotropiumbromid können zum Waschen der erhaltenen Tiotropiumbromid-monohydrat-Kristalle 0,1 bis 1,0 L, bevorzugt 0,2 bis 0,5 L, besonders bevorzugt etwa 0,3 L Lösemittel Verwendung finden. Gegebenenfalls kann der Waschritt wiederholt durchgeführt werden.

Das erhaltene Produkt wird im Vakuum oder mittels erwärmter Umluft bis zum Erreichen eines Wassergehalts von 2,5 - 4,0 % getrocknet.

Das so erhaltene kristalline Tiotropiumbromid-Monohydrat wird in den nachfolgend beschriebenen Mahlprozess (Mikronisierung) eingesetzt. Zur Durchführung dieses Prozesses können gängige Mühlen zum Einsatz gelangen. Bevorzugt wird die Mikronisierung dabei unter Feuchtigkeitsausschluß durchgeführt, besonders bevorzugt unter Einsatz eines entsprechenden Inertgases, wie beispielsweise Stickstoff. Als besonders bevorzugt hat sich die Verwendung von Luftstrahlmühlen erwiesen, in denen die Zerkleinerung des Mahlguts durch Aufeinenderprallen der Partikel miteinender sowie Aufprall der Partikel auf die Wände des Mahlbehälters erfolgt. Als Mahlgas gelangt erfindungsgemäß bevorzugt Stickstoff zur Anwendung. Das Mahlgut wird mittels des Mahlgases unter spezifischen Drücken (Mahldruck) gefördert. Im Rahmen der vorliegenden Erfindung wird der Mahldruck üblicherweise auf einen Wert zwischen etwa 2 und etwa 8 bar, bevorzugt zwischen etwa 3 und etwa 7 bar, besonders bevorzugt zwischen etwa 3,5 und etwa 6,5 bar eingestellt. Der Eintrag des Mahlgutes in die Luftstrahlmühle erfolgt mittels des Speisegases unter spezifischen Drücken (Speisedruck). Im Rahmen der vorliegenden Erfindung hat sich eine Speisedruck zwischen etwa 2 und etwa 8 bar, bevorzugt zwischen etwa 3 und etwa 7 bar, besonders bevorzugt zwischen etwa 3,5 und etwa 6 bar bewährt. Als Speisegas gelangt vorzugsweise ebenfalls ein Inertgas, besonders bevorzugt ebenfalls Stickstoff zur Anwendung. Die Zufuhr des Mahlguts (kristallines Tiotropiumbromidmonohydrat) kann dabei in einer Förderräte von etwa 5 - 35 g/min, vorzugsweise mit etwa 10-30 g/min erfolgen.

Beispielsweise und ohne den Gegenstand der Erfindung darauf zu beschränken, hat sich als eine mögliche Ausführungsform einer Luftstrahlmühle das folgende Gerät bewährt: 2-Zoll Microniser mit Mahlring 0,8 mm-Bohrung, Firma

Sturtevant Inc., 348 Circuit Street, Hanover, MA 02239, USA. Unter Verwendung dieses Geräts wird der Mahlprozess vorzugsweise mit folgenden Mahlparametern durchgeführt: Mahldruck: etwa 4,5 - 6,5 bar; Speisedruck: etwa 4,5 - 6,5 bar: Zufuhr des Mahlguts: etwa 17 - 21 g/min.

Das so erhaltene Mahlgut wird anschließend unter den nachfolgend genannten spezifischen Bedingungen weiterverarbeitet. Hierzu wird das Mikronisat bei einer Temperatur von 15 - 40 °C, vorzugsweise 20 - 35 °C, besonders bevorzugt bei 25 - 30 °C Wasserdampf einer relativen Feuchte von wenigstens 40% ausgesetzt. Vorzugsweise wird die Feuchte auf einen Wert von 50 - 95% r.F., bevorzugt auf 60 - 90 % r.F., besonders bevorzugt auf 70 - 80 % r.F. eingestellt. Unter relativer Feuchte (r.F.) wird im Rahmen der vorliegenden Erfindung der Quotient des Partialdruckes des Wasserdampfs und des Dampfdruckes des Wassers bei der betreffenden Temperatur verstanden. Vorzugsweise wird das aus vorstehend beschriebenem Mahlprozess erhältliche Mikronisat den oben genannten Raumbedingungen wenigstens über einen Zeitraum von 6 Stunden ausgesetzt. Bevorzugt wird das Mirkonisat den genannten Raumbedingungen allerdings für etwa 12 bis etwa 48 Stunden, vorzugsweise etwa 18 bis etwa 36 Stunden, besonders bevorzugt etwa 20 bis etwa 28 Stunden ausgesetzt.

Das gemäß vorstehender Vorgehensweise erhältliche erfindungsgemäße Mikronisat des Tiotropiumbromids weist eine charakteristische Partikelgröße $X_{50}$ von zwischen 1,0 μm und 3,5 μm, bevorzugt zwischen 1,1μm und 3,3 μm, besonders bevorzugt zwischen 1,2 μm und 3,0μm und $Q_{(5.8)}$ von größer 60%, bevorzugt größer 70 %, besonders bevorzugt größer 80% auf. Dabei bezeichnet der Kennwert $X_{50}$ den Medianwert der Teilchengröße, unterhalb derer 50% der Teilchenmenge bezüglich der Volumenverteilung der einzelnen Teilchen liegt. Der Kennwert $Q_{(5.8)}$ entspricht der Teilchenmenge der Partikel, die bezogen auf die Volumenverteilung der Partikel unterhalb von 5.8 μm liegt. Die Partikelgrößen wurden im Rahmen der vorliegenden Erfindung mittels Laserbeugung (Fraunhoferbeugung) bestimmt. Detailliertere Angaben dazu sind den experimentellen Beschreibungen der Erfindung zu entnehmen.

Ebenso charakteristisch für das erfindungsgemäße Tiotropium-Mikronisat, das nach obigem Prozeß dargestellt wurde, sind Spezifische Oberflächenwerte im Bereich zwischen 2 $m^2$/g und 5 $m^2$/g, im besonderen Maße Werte zwischen 2,5 $m^2$/g und 4,5 $m^2$/g und in besonders herausragendem Maße zwischen 3,0 $m^2$/g und 4,0 $m^2$/g.

Die Durchführung des Prozesses führt zum erfindungsgemäßen Mikronisat des Tiotropiumbromids, welches durch spezifische Lösungswärmen gekennzeichnet ist. Diese weisen vorzugsweise einen Wert von größer 65 Ws/g, bevorzugt größer 71 Ws/g auf. Besonders bevorzugt übersteigt der Wert der Lösungswärme des erfindungsgemäßen Mikronisats den Betrag von 74 Ws/g. Detaillierte Angaben zur Bestimmung der Lösungsenthalpien sind den experimentellen Beschreibungen der Erfindung zu entnehmen.

Das Tiotropiumbromid-mikronisat, das mit Hilfe des obigen Verfahrens gewonnen werden kann, zeichnet sich ferner dadurch aus, daß der Wassergehalt des Mikronisats zwischen etwa 1 % und etwa 4,5 %, bevorzugt zwischen etwa 1,4% und 4,2% besonders bevorzugt zwischen etwa 2,4% und 4,1% liegt. Erfingdungsgemäß besonders bevorzugtes Tiotropiumbromid-mikronisat ist dadurch gekennzeichnet, daß der Wassergehalt des Mikronisats zwischen etwa 2,6 % und etwa 4,0 %, besonders bevorzugt zwischen etwa 2,8% und 3,9% besonders bevorzugt zwischen etwa 2,9% und 3,8% liegt.

Ein Aspekt der vorliegenden Erfindung betrifft Tiotropiumbromidmikronisat, welches die vorstehend genannten Charakteristika aufweist.

Im Rahmen der vorliegenden Erfindung ist, soweit nichts anderes angegeben, eine Bezugnahme auf Tiotropiumbromid-mikronisat als Bezugnahme auf jenes kristalline Mikronisat des Tiotropiumbromids zu verstehen, welches die vorstehend genannten Charakteristika aufweist und welches gemäß dem zuvor beschriebenen
Verfahren (Mikronisierung und anschließende Weiterbehandlung gemäß den zuvor beschriebenen Parametern) erhältlich ist.

Ein weiterer Aspekt der vorliegenden Erfindung betrifft aufgrund der pharmazeutischen Wirksamkeit des erfindungsgemäßen Mikronisats die Verwendung des erfindungsgemäßem Tiotropiumbromid-mikronisats als Arzneimittel.

Ein weiterer Aspekt der vorliegenden Erfindung betrifft Inhalationspulver gekennzeichnet durch einen Gehalt an erfindungsgemäßem Tiotropiumbromidmikronisat.

Aufgrund der anticholinergen Wirksamkeit von Tiotropiumbromid zielt ein weiterer Aspekt der vorliegenden Erfindung auf die Verwendung des erfindungsgemäßen Tiotropiumbromid-mikronisats zur Herstellung eines Arzneimittels zur Behandlung von Erkrankungen, in denen die Applikation eines Anticholinergikums einen therapeutischen Nutzen entfalten kann. Bevorzugt ist die entsprechende Verwendung zur Herstellung eines Arzneimittels zur Behandlung von Asthma oder COPD.

Das gemäß dem erfindungsgemäßen Verfahren zugängliche Tiotropiumbromid-Mikronisat ist in herausragender Weise zur Darstellung pharmazeutischer Formulierungen geeignet. Besonders bevorzugt kann es zur Herstellung von Inhalationspulvern Verwendung finden.

Dementsprechend zielt die vorliegende Erfindung auf Inhalationspulver enthaltend wenigstens etwa 0,03 %, vorzugsweise unter 5 %, besonders bevorzugt unter 3 % des nach vorstehend beschriebenem Verfahren erhältlichen Tiotropiumbromid-mikronisats im Gemisch mit einem physiologisch unbedenklichen Hilfsstoff, dadurch gekennzeichnet, daß der Hilfsstoff aus einem Gemisch von gröberem Hilfsstoff mit einer mittleren Teilchengröße von 15 bis 80μm und feinerem

Hilfststoff mit einer mittleren Teilchengröße von 1 bis 9 µm besteht, wobei der Anteil von feinerem Hilfsstoff an der Gesamthilfsstoffmenge 1 bis 20% beträgt.

Bei den vorstehend genannten prozentualen Angaben, handelt es sich um Gewichtsprozent.

Erfindungsgemäß bevorzugt sind Inhalationspulver, die etwa 0,05 bis etwa 1%, bevorzugt etwa 0,1 bis etwa 0,8%, besonders bevorzugt etwa 0,2 bis etwa 0,5% Tiotropiumbromid-mikronisat enthalten, welches nach vorstehend beschriebenem Verfahren erhältlich ist und die kennzeichnenden Charkteristika des erfindungsgemäß erhältlichen Mikronisats aufweist.

Die das erfindungsgemäße Mikronisat enthaltenden Inhalationspulver sind bevorzugt dadurch gekennzeichnet, daß der Hilfsstoff aus einem Gemisch von gröberem Hilfsstoff mit einer mittleren Teilchengröße von 17 bis 50µm, besonders bevorzugt von 20 bis 30µm und feinerem Hilfststoff mit einer mittleren Teilchengröße von 2 bis 8µm, besonders bevorzugt von 3 bis 7µm besteht. Dabei wird unter der mittleren Teilchengröße im hier verwendeten Sinne der 50%-Wert aus der Volumenverteilung gemessen mittels Laserbeugungnach der Trockendispersionsmethode verstanden. Bevorzugt sind Inhalationspulver bei denen der Anteil von feinerem Hilfsstoff an der Gesamthilfsstoffmenge 3 bis 15%, besonders bevorzugt 5 bis 10% beträgt.

Wird im Rahmen der vorliegenden Erfindung auf die Bezeichnung Gemisch Bezug genommen, so ist hierbei stets eine Mischung zu verstehen, die durch Mischen zuvor klar definierter Komponenten erhalten wurde. Entsprechend sind beispielsweise als Hilfsstoffgemisch aus gröberen und feineren Hilfsstoffanteilen nur solche Gemische zu verstehen, die durch Mischen einer gröberen Hilfsstoffkomponente mit einer feineren Hilfsstoffkomponente erhalten werden.

Die gröberen und feineren Hilfsstoffanteile können aus dem chemisch gleichen oder aus chemisch verschiedenen Substanzen bestehen, wobei Inhalationspulver, bei denen der gröbere Hilfsstoffanteil und der feinere Hilfsstoffanteil aus der selben chemischen Verbindung bestehen bevorzugt sind.

Als physiologisch unbedenkliche Hilfsstoffe, die zur Darstellung der das erfindungsgemäße Mikronisat enthaltenden Inhalationspulver zur Anwendung gelangen können seien beispielsweise genannt: Monosaccharide (z.B. Glucose oder Arabinose), Disaccharide (z.B. Lactose, Saccharose, Maltose oder Trehalose), Oligo- und Polysaccharide (z.B. Dextrane), Polyalkohole (z.B. Sorbit, Mannit, Xylit), Salze (z.B. Natriumchlorid, Calciulcarbonat) oder Mischungen dieser Hilfsstoffe miteinander. Bevorzugt gelangen Mono- oder Disaccharide zur Anwendung, wobei die Verwendung von Lactose, Glucose oder Trehalose, bevorzugt Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.

Die das erfindungsgemäße Mikronisat enthaltenden Inhalationspulver können beispielsweise mittels Inhalatoren appliziert werden, die eine einzelne Dosis aus einem Vorrat mittels einer Meßkammer (z.B. gemäß US 4570630A) oder über andere apparative Vorrichtungen (z.B. gemäß DE 36 25 685 A) dosieren. Vorzugsweise werden die Inhalationspulver allerdings in Kapseln abgefüllt (zu sogenannten Inhaletten), die in Inhalatoren wie beispielsweise in der WO 94/28958 beschrieben, zur Anwendung gelangen.

Sollen die erfindungsgemäßen Inhalationspulver im Sinne der vorstehend gennannten bevorzugten Anwendung in Kapseln (Inhaletten) sowie sonstigen Verpackungsformen, die Einzeldosen anbieten, abgefüllt werden, bieten sich Füllmengen von 1 bis 15mg, bevorzugt 3 bis 10mg, höchst bevorzugt von 4 bis 6mg Inhalationspulver pro Kapsel an.

Die das erfindungsgemäße Tiotropioumbromid-mikronisat enthaltenden Inhalationspulver sind durch ein hohes Maß an Homogenität im Sinne der Einzeldosierungsgenauigkeit gekennzeichnet. Diese liegt in einem Bereich von < 8%, bevorzugt < 6% , besonders bevorzugt < 4%.

Die das erfindungsgemäße Tiotropiumbromidmikronisat enthaltenden Inhalationspulver sind gemäß der nachfolgend beschriebenen Vorgehensweise erhältlich.

Nach Einwaage der Ausgangsmaterialien erfolgt zunächst die Fertigung der Hilfsstoffmischung aus den definierten Fraktionen des gröberen Hilfsstoffs und des feineren Hilfsstoffs. Anschließend erfolgt die Herstellung der erfindungsgemäßen Inhalationspulver aus der Hilfsstoffmischung und dem Wirkstoff. Soll das Inhalationspulver mittels Inhaletten in hierzu geeigneten Inhalatoren appliziert werden, schließt sich der Herstellung der Inhalationspulver die Fertigung der pulverhaltigen Kapseln an.

Bei den nachstehend beschriebenen Herstellverfahren werden die genannten Komponenten in den Gewichtsanteilen eingesetzt, wie sie in den zuvor beschriebenen Zusamensetzungen der erfindungsgemäßen Inhalationspulver beschrieben wurden.

Die Herstellung der erfindungsgemäßen Inhaltionspulver erfolgt durch Mischen der gröberen Hilfsstoffanteile mit den feineren Hilfststoffanteilen und andschließendem Mischen der so erhaltenen Hilfsstoffgemische mit dem Wirkstoff.

Zur Herstellung der Hilfsstoffmischung werden die gröberen und feineren Hilfsstoffanteile in einen geeigneten Mischbehälter eingebracht. Die Zugabe der beiden Komponenten erfolgt vorzugsweise über einen Siebgranulator mit einer Maschenweite von 0,1 bis 2 mm, besonders bevorzugt 0,3 bis 1 mm, höchst bevorzugt 0,3 bis 0,6 mm. Vorzugsweise wird der gröbere Hilfsstoff vorgelegt und anschließend der feinere Hilfsstoffanteil in den Mischbehälter eingebracht. Bevorzugt erfolgt bei diesem Mischverfahren die Zugabe der beiden Komponenten portionsweise, wobei ein Teil des gröberen Hilfsstoffs zunächst vorgelegt und anschließend abwechselnd feinerer und gröberer Hilfsstoff zugegeben wird.

Besonders bevorzugt ist bei der Herstellung der Hilfsstoffmischung das abwechselnde, schichtweise Einsieben der beiden Komponenten. Vorzugsweise erfolgt das Einsieben der beiden Komponenten abwechselnd in je 15 bis 45, besonders bevorzugt in je 20 bis 40 Schichten. Der Mischvorgang der beiden Hilfsstoffe kann bereits während der Zugabe der beiden Komponenten erfolgen. Vorzugsweise wird allerdings erst nach dem schichtweisen Einsieben der beiden Bestandteile gemischt.

Nach Herstellung der Hilfsstoffmischung werden diese und der Wirkstoff, das erfindungsgemäße Tiotropiumbromid-mikronisat, in einen geeigneten Mischbehälter eingebracht. Der verwendete Wirkstoff weist eine mittlere Teilchengröße von 0,5 bis 10μm, vorzugsweise von 1 bis 6μm, besonders bevorzugt von 1,5 bis 5μm auf. Die Zugabe der beiden Komponenten erfolgt vorzugsweise über einen Siebgranulator mit einer Maschenweite von 0,1 bis 2 mm, besonders bevorzugt 0,3 bis 1 mm, höchst bevorzugt 0,3 bis 0,6 mm. Vorzugsweise wird die Hilfsstoffmischung vorgelegt und anschließend der Wirkstoff in den Mischbehälter eingebracht. Bevorzugt erfolgt bei diesem Mischverfahren die Zugabe der beiden Komponenten portionsweise. Besonders bevorzugt ist bei der Herstellung der Hilfsstoffmischung das abwechselnde, schichtweise Einsieben der beiden Komponenten. Vorzugsweise erfolgt das Einsieben der beiden Komponenten abwechselnd in je 25 bis 65, besonders bevorzugt in je 30 bis 60 Schichten. Der Mischvorgang der Hilfsstoffmischung mit dem Wirkstoff kann bereits während der Zugabe der beiden Komponenten erfolgen. Vorzugsweise wird allerdings erst nach dem schichtweisen Einsieben der beiden Bestandteile gemischt.

Die so erhaltene Pulvermischung kann gegebenenfalls erneut ein- oder mehrfach über einen Siebgranulator gegeben und jeweils anschließend einem weiteren Mischvorgang unterworfen werden.

Ein weiterer Aspekt der vorliegenden Erfindung betrifft ein Inhaltionspulver, welches das erfindungsgemäße Tiotropiumbromid-Mikronisat enthält und gemäß der voranstehend beschriebenen Vorgehensweisen erhältlich ist.

Die folgenden, detaillierten experimentellen Ausführungen dienen einer weitergehenden Erläuterung der vorliegenden Erfindung, ohne den Umfang der Erfindung allerdings auf die nachfolgenden beispielhaften Ausführungsformen zu beschränken.

## Experimenteller Teil

### A) Darstellung von kristallinem Tiotropiumbromid-monohydrat

In einem geeigneten Reaktionsgefäß werden in 25,7 kg Wasser 15,0 kg Tiotropiumbromid eingetragen, welches beispielsweise gemäß der in der Europäischen Patentanmeldung EP 418 716 A1 offenbarten experimentellen Vorgehensweise erhältlich ist. Die Mischung wird auf 80-90°C erhitzt und bei gleichbleibender Temperatur solange gerührt, bis eine klare Lösung entsteht. Aktivkohle (0,8 kg), wasserfeucht, wird in 4,4 kg Wasser aufgeschlämmt, diese Mischung in die Tiotropiumbromid-haltige Lösung eingetragen und mit 4,3 kg Wasser nachgespült. Die so erhaltene Mischung wird wenigstens 15 min bei 80-90°C gerührt und anschließend über einen beheizten Filter in einen auf 70°C Manteltemperatur vorgewärmten Apparat filtriert. Der Filter wird mit 8,6 kg Wasser nachgespült. Der Apparateinhalt wird mit 3-5°C pro 20 Minuten auf eine Temperatur von 20-25°C abgekühlt. Mit Kaltwasserkühlung wird der Apparat auf 10-15°C weiter abgekühlt und die Kristallisation durch mindestens einstündiges Nachrühren vervollständigt. Das Kristallisat wird über einen Nutschentrockner isoliert, der isolierte Kristallbrei mit 9 L kaltem Wasser (10-15°C) und kaltem Aceton (10-15°C) gewaschen. Die erhaltenen Kristalle werden bei 25°C über 2 Stunden im Stickstoffstrom getrocknet.
Ausbeute : 13,4 kg Tiotropiumbromid-monohydrat (86 % d. Th.)

### B) Charakterisierung des kristallinen Tiotropiumbromid-monohydrats

Das gemäß vorstehend beschriebener Vorgehensweise erhältliche Tiotropiumbromid-monohydrat wurde einer Untersuchung mittels DSC (Differential Scanning Calorimetry) unterworfen. Das DSC- diagramm weist zwei charakteristische Signale auf. Das erste, relativ breite, endotherme Signal zwischen 50-120°C ist auf die Entwässerung des Tiotropiumbomid-monohydrats zur wasserfreien Form zurückzuführen. Das zweite, relativ scharfe, endotherme Maximum bei 230 ± 5°C ist dem Schmelzen der Substanz zuzuordnen. Diese Daten wurden mittels eines Mettler DSC 821 erhalten und mit dem Mettler Software-Paket STAR ausgewertet. Die Daten wurden bei einer Heizrate von 10 K/min erhoben. Da die Substanz unter Zersetzung schmilzt (= inkongruenter Schmelzvorgang), hängt der beobachtete Schmelzpunkt sehr von der Heizrate ab. Bei geringeren Heizraten wird der Schmelz-/Zersetzungsvorgang bei deutlich niedrigeren Temperaturen beobachtet, so zum Beispiel mit einer Heizrate von 3 K/min bei 220 ± 5 °C. Es kann außerdem vorkommen, daß der Schmelzpeak aufgespalten vorliegt. Die Aufspaltung tritt umso stärker auf, je geringer die Heizrate im DSC-Experiment ist.

Das kristalline Tiotropiumbromid-monohydrat wurde mittels IR-Spektroskopie charakterisiert. Die Daten wurden mittels eines Nicolet FTIR Spektrometers erhoben und mit dem Nicolet Softwarepaket OMNIC, version 3.1 ausgewertet. Die Messung wurde mit 2,5μmol Tiotropiumbromid-monohydrat in 300 mg KBr durchgeführt.

Tabelle 1 faßt einige der wesentlichen Banden des IR-Spektrums zusammen.

Tabelle 1: Zuordnung von spezifischen Banden

| Wellenzahl ($cm^{-1}$) | Zuordnung | Schwingungstyp |
|---|---|---|
| 3570,3410 | O-H | Streckschwingung |
| 3105 | Aryl C-H | Streckschwingung |
| 1730 | C=O | Streckschwingung |
| 1260 | Epoxid C-O | Streckschwingung |
| 1035 | Ester C-OC | Streckschwingung |
| 720 | Thiophen | Ringschwingung |

Das kristalline Tiotropiumbromid-monohydrat wurde mittels Röntgenstrukturanalyse charakterisiert. Die Röntgenbeugungsintensitätsmessungen wurden auf einem AFC7R- 4-Kreisdiffraktometer (Rigaku) unter Verwendung von monochromatisierter Kupfer K$\alpha$-Strahlung durchgeführt. Die Strukturlösung und Verfeinerung der Kristallstruktur erfolgte mittels Direkter Methoden (Programm SHELXS86) und FMLQ-Vefeinerung (Programm TeXsan). Experimentelle Details zur Kristallstruktur, Strukturlösung und -verfeinerung sind in Tabelle 2 zusammengefaßt.

Tabelle 2: Experimentelle Daten zur Kristallstrukturanalyse von Tiotropiumbromidmonohydrat.

| | |
|---|---|
| A. Kristalldaten | |
| Empirische Formel | $[C_{19}H_{22}NO_4S_2]$ Br · $H_20$ |
| Formelgewicht | 472.43 + 18.00 |
| Kristallfarbe, -gestalt | farblos, prismatisch |
| Kristallabmessungen | 0.2 x 0.3 x 0.3 mm |
| Kristallsystem | monoklin |
| Gittertyp | primitv |
| Raumgruppe | P $2_1$/n |
| Gitterkonstanten | a = 18.0774 Å,<br>b = 11.9711 Å<br>c = 9.9321 Å<br>$\beta$ = 102.691°<br>V = 2096.96 $Å^3$ |
| Formeleinheiten pro Elementarzelle | 4 |
| B. Intensitätsmessungen | |
| Diffraktometer | Rigaku AFC7R |
| Röntgengenerator | Rigaku RU200 |
| Wellenlänge | $\lambda$ = 1.54178Å (monochromatisierte Kupfer K$\alpha$-Strahlung) |
| Strom, Spannung | 50kV, 100mA |
| Take-off Winkel | 6° |
| Kristallmontage | wasserdampfgesättigte Kapillare |
| Kristall-Detektor-Abstand | 235mm |
| Detektor Öffnung | 3.0 mm vertikal und horizontal |
| Temperatur | 18° |
| Bestimmung der Gitterkonstanten | 25 Reflexe (50.8° < 20 < 56.2°) |
| Scan Typ | $\omega$ - 2$\Theta$ |
| Scan Geschwindigkeit | 8.0 32.0°/min in w |
| Scan Breite | (0.58 + 0.30 tan $\Theta$) ° |
| 2$\Theta$max | 120° |
| Messungen | 5193 |
| Unabhängige Reflexe | 3281 ( Rint=0.051) |
| Korrrekturen | Lorentz-Polarisation<br>Absorption |

(fortgesetzt)

| | |
|---|---|
| B. Intensitätsmessungen | |
| | (Transmissionsfaktoren 0.56 - 1.00) |
| | Kristall-decay 10.47% Abfall |
| C. Verfeinerung | |
| Reflexe (I > 3σI) | 1978 |
| Variable | 254 |
| Verhältnis Reflexe/Parameter | 7.8 |
| R-Werte: R, Rw | 0.062, 0.066 |

Die durchgeführte Röntgenstrukturanlyse ergab, daß kristallines Toptropiumbromidhydrat eine einfache monoklinische Zelle mit folgenden Dimensionen aufweist: a = 18.0774 Å, b = 11.9711 Å, c = 9.9321 Å, β = 102.691 °, V = 2096.96 $Å^3$.

Durch die vorstehende Röntgenstrukturanalyse wurden die in Tabelle 3 beschriebenen Atomkoordinaten bestimmt:

Tabelle 3: Koordinaten

| Atom | x | y | z | u (eq) |
|---|---|---|---|---|
| Br(1) | 0.63938(7) | 0.0490(1) | 0.2651(1) | 0.0696(4) |
| S(1) | 0.2807(2) | 0.8774(3) | 0.1219(3) | 0.086(1) |
| S(2) | 0.4555(3) | 0.6370(4) | 0.4214(5) | 0.141(2) |
| O(1) | 0.2185(4) | 0.7372(6) | 0.4365(8) | 0.079(3) |
| O(2) | 0.3162(4) | 0.6363(8) | 0.5349(9) | 0.106(3) |
| O(3) | 0.3188(4) | 0.9012(5) | 0.4097(6) | 0.058(2) |
| O(4) | 0.0416(4) | 0.9429(6) | 0.3390(8) | 0.085(3) |
| O(5) | 0.8185(5) | 0.0004(8) | 0.2629(9) | 0.106(3) |
| N(1) | 0.0111(4) | 0.7607(6) | 0.4752(7) | 0.052(2) |
| C(1) | 0.2895(5) | 0.7107(9) | 0.4632(9) | 0.048(3) |
| C(2) | 0.3330(5) | 0.7876(8) | 0.3826(8) | 0.048(3) |
| C(3) | 0.3004(5) | 0.7672(8) | 0.2296(8) | 0.046(3) |
| C(4) | 0.4173(5) | 0.7650(8) | 0.4148(8) | 0.052(3) |
| C(5) | 0.1635(5) | 0.6746(9) | 0.497(1) | 0.062(3) |
| C(6) | 0.1435(5) | 0.7488(9) | 0.6085(9) | 0.057(3) |
| C(7) | 0.0989(6) | 0.6415(8) | 0.378(1) | 0.059(3) |
| C(8) | 0.0382(5) | 0.7325(9) | 0.3439(9) | 0.056(3) |
| C(9) | 0.0761(6) | 0.840(1) | 0.315(1) | 0.064(3) |
| C(10) | 0.1014(6) | 0.8974(8) | 0.443(1) | 0.060(3) |
| C(11) | 0.0785(5) | 0.8286(8) | 0.5540(9) | 0.053(3) |
| C(12) | -0.0632(6) | 0.826(1) | 0.444(1) | 0.086(4) |
| C(13) | -0.0063(6) | 0.6595(9) | 0.554(1) | 0.062(3) |
| C(14) | 0.4747(4) | 0.8652(9) | 0.430(1) | 0.030(2) |
| C(15) | 0.2839(5) | 0.6644(9) | 0.1629(9) | 0.055(3) |
| C(16) | 0.528(2) | 0.818(2) | 0.445(2) | 0.22(1) |
| C(17) | 0.5445(5) | 0.702(2) | 0.441(1) | 0.144(6) |
| C(18) | 0.2552(6) | 0.684(1) | 0.019(1) | 0.079(4) |
| C(19) | 0.2507(6) | 0.792(1) | -0.016(1) | 0.080(4) |
| H(1) | -0.0767 | 0.8453 | 0.5286 | 0.102 |
| H(2) | -0.0572 | 0.8919 | 0.3949 | 0.102 |
| H(3) | -0.1021 | 0.7810 | 0.3906 | 0.102 |
| H(4) | -0.0210 | 0.6826 | 0.6359 | 0.073 |
| H(5) | -0.0463 | 0.6178 | 0.4982 | 0.073 |

(fortgesetzt)

| Atom | x | y | z | u (eq) |
|---|---|---|---|---|
| H(6) | 0.0377 | 0.6134 | 0.5781 | 0.073 |
| H(7) | 0.1300 | 0.7026 | 0.6770 | 0.069 |
| H(8) | 0.1873 | 0.7915 | 0.6490 | 0.069 |
| H(9) | 0.1190 | 0.6284 | 0.2985 | 0.069 |
| H(10) | 0.0762 | 0.5750 | 0.4016 | 0.069 |
| H(11) | 0.1873 | 0.6082 | 0.5393 | 0.073 |
| H(12) | -0.0025 | 0.7116 | 0.2699 | 0.066 |
| H(13) | 0.1084 | 0.8383 | 0.2506 | 0.075 |
| H(14) | 0.1498 | 0.9329 | 0.4626 | 0.071 |
| H(15) | 0.0658 | 0.8734 | 0.6250 | 0.063 |
| H(16) | 0.2906 | 0.5927 | 0.2065 | 0.065 |
| H(17) | 0.2406 | 0.6258 | -0.0469 | 0.094 |
| H(18) | 0.2328 | 0.8191 | -0.1075 | 0.097 |
| H(19) | 0.4649 | 0.9443 | 0.4254 | 0.037 |
| H(20) | 0.5729 | 0.8656 | 0.4660 | 0.268 |
| H(21) | 0.5930 | 0.6651 | 0.4477 | 0.165 |
| H(22) | 0.8192 | -0.0610 | 0.1619 | 0.084 |
| H(23) | 0.7603 | 0.0105 | 0.2412 | 0.084 |

x, y, z: fraktionelle Koordinaten;
U(eq) mittlere quadratische Amplitude atomarer Bewegung im Kristall;

## C) Darstellung des erfindungsgemäßen Tiotropiumbromid-mikronisats

Das gemäß vorstehend beschriebener Vorgehensweise erhältliche Tiotropiumbromid-monohydrat wird mit einer Luftstrahlmühle vom Typ 2-Zoll Microniser mit Mahlring 0,8 mm-Bohrung, Firma Sturtevant Inc., 348 Circuit Street, Hanover, MA 02239, USA mikronisiert. Unter Verwendung von Stickstoff als Mahlgas werden dabei beispielsweise die folgenden Mahlparameter eingestellt:

Mahldruck: 5,5 bar; Speisedruck: 5,5 bar;
Zufuhr (des kristallinen Monohydrats) bzw. Fließgeschwindigkeit: 19 g/min.

Das erhaltene Mahlgut wird anschließend auf Hordenblechen in einer Schichtdicke von etwa 1 cm ausgebreitet und für 24 - 24,5 Stunden den folgenden Klimabedingungen unterworfen: Temperatur: 25 - 30 °C; Relative Feuchte: 70-80%.

## D) Meßtechniken zur Charakterisierung des erfindungsgemäßen Tiotropiumbromid-mikronisats

Die in der Beschreibung genannten, das erfindungsgemäße Tiotropiumbromidmikronisat charakterisierenden Parameter wurden gemäß den nachfolgend beschriebenen Meßtechniken und Methoden erhalten:

### D.1) Bestimmung des Wassergehalts nach Karl-Fischer (Tiotropium Bromid):

| | |
|---|---|
| Titrator | Typ Mettler DL 18 mit |
| Kalibriersubstanz: | Dinatriumtartratdihydrat |
| Titrant: | Hydranal-Titrant 5 (Riedel-deHaen) |
| Lösungsmittel: | Hydranal Solvent (Riedel-deHaen) |

Meßmethode:

| | |
|---|---|
| Probenmenge: | 50 - 100 mg |
| Rührzeit: | 60 s |

Die Rührzeit vor Beginn der Titration dient dazu, die vollständige Auflösung der Probe zu gewährleisten.

Der Wassergehalt der Probe wird vom Gerät in Prozent berechnet und ausgegeben.

**D.2) Partikelgrößenbestimmung mittels Laserbeugung (Fraunhoferbeugung)** Meßmethode:

Zur Bestimmung der Partikelgröße wird das Pulver mittels Dispergiereinheit einem Laserbeugungs-Spektrometer zugeführt.

| | |
|---|---|
| Meßgerät: | Laser-Beugungs-Spektrometer (HELOS),Fa. Sympatec |
| Software: | WINDOX Version 3.3/REL 1 |
| Dispergiereinheit: | RODOS / Dispergierdruck: 3 bar |

Geräteparameter:

| | |
|---|---|
| Detektor: | Multielementdetektor (31 halbkreisförmige Ringe) |
| Methode: | Luft-Dispergierung |
| Brennweite: | 100 mm |
| Messbereich: | RS 0,5/0,9 - 175 μm |
| Auswertemodus: | HRLD-Mode |

Rodos Trockendispergierer:

| | |
|---|---|
| Injektor: | 4 mm |
| Druck: | 3 bar |
| Injektor-Unterdruck: | (~ 100 mbar) |
| Absaugung: | Nilfilsk (Vorlauf 5 s) |
| Dosierer: | Vibri |
| Förderrate: | 40 % (manuelle Steigerung bis 100 %) |
| Betthöhe: | 2 mm |
| Drehzahl: | 0 |

**D.3) Bestimmung der Spezifischen Oberfläche (1-Pkt.-B.E.T.-Methode):** Meßmethode:

Die Bestimmung der spezifischen Oberfläche erfolgt, indem die Pulverprobe einer Stickstoff/Heliumatmosphäre bei unterschiedlichen Drücken ausgesetzt wird. Durch Abkühlung der Probe erfolgt eine Kondensation der Stickstoffmolekülen auf der Oberfläche der Partikel. Die kondensierte Stickstoffmenge wird über die Änderung der thermischen Leitfähigkeit des Stickstoff/Heliumgemisches bestimmt und die Oberfläche der Probe über den Flächenbedarf von Stickstoff bestimmt. Über diesen Wert und die Probeneinwaage wird die spezifische Oberfläche berechnet.

Geräte und Materialien:

| | |
|---|---|
| Messgerät: | Monosorb, Fa. Quantachrome |
| Ausheizgerät: | Monotektor, Fa. Quantachrome |
| Mess- und Trockengas: | Stickstoff (5.0) / Helium (4.6) 70/30, Fa. Messer Griesheim |
| Adsorbat: | Stickstoff 30%ig in Helium |
| Kältemittel: | flüssiger Stickstoff |
| Messzelle: | mit Kapillarrohr, Fa. W. Pabisch GmbH&Co.KG |
| Kalibrierspritze; | 1000 μl, Fa. Precision Sampling Corp. |
| Analysenwaage: | R 160 P, Fa. Satorius |

Berechnung der spezifischen Oberfläche:

Die Messwerte werden vom Gerät in [$m^2$] angezeigt und werden i.d.R. in [$cm^2/g$] auf die Einwaage (Trockenmasse) umgerechnet:

$$A_{spez} = \frac{MW * 10000}{m_{tr}}$$

| | |
|---|---|
| $A_{spez}$ | = spezifische Oberfläche [$cm^2/g$] |
| MW | = Messwert [$m^2$] |
| $m_{tr}$ | = Trockenmasse [g] |
| 10000 | = Umrechnungsfaktor [$cm^2/m^2$] |

**D.4) Bestimmung der Lösungswärme (Lösungsenthalpie) $E_c$:**

Die Bestimmung der Lösungsenthalpie erfolgt mittels eines Lösungskalorimeter *2225 Precision Solution Calorimeter* der Fa. Thermometric .

Die Lösungswärme wird anhand der - infolge des Löseprozesses - auftretenden Temperaturänderung und der aus der Basislinie berechneten systembedingten Temperaturänderung berechnet.

Vor und nach dem Ampullenbruch wird jeweils eine elektrische Kalibrierung mit einem integrierten Heizwiderstand genau bekannter Leistung durchgeführt. Hierbei wird eine bekannte Wärmeleistung über einen festgelegten Zeitraum an das System abgegeben und der Temperatursprung ermittelt.

Methoden- und Geräteparameter:

| | |
|---|---|
| Lösungskalorimeter: | 2225 Precision Solution Calorimeter, Fa. Thermometric |
| Reaktionszelle: | 100 ml |
| Thermistorwiderstand: | 30,0 kΩ (bei 25 °C) |
| Rührergeschwindigkeit: | 600 U/min |
| Thermostat: | Thermostat des 2277 Thermal Activity Monitor TAM, Fa Thermometric |
| Temperatur: | 25 °C ± 0.0001 °C (über 24h) |
| Meßampullen: | Crushing ampoules 1 ml, Fa. Thermometric |
| Dichtung: | Silikonstopfen und Bienenwachs, Fa. Thermometric |
| Einwaage: | 40 bis 50 mg |
| Lösemittel: | Wasser, chemisch rein |
| Volumen Lösemittel: | 100 ml |
| Badtemperatur: | 25°C |
| Temperaturauflösung: | High |
| Starttemperatur: | -40mK (± 10mK) temperature-offset |
| Interface: | 2280-002 TAM accessory interface 50 Hz, Fa. Thermometric |
| Software: | SolCal V 1.1 für WINDOWS |
| Auswertung: | Automatische Auswertung mit Menüpunkt CALCULATION/ ANALYSE EXPERIMENT. (Dynamik der Basislinie ; Kalibrierung nach dem Ampullenbruch). |

Elektrische Kalibrierung:

Die elektrische Kalibrierung erfolgt während der Messung, einmal vor und einmal nach dem Ampullenbruch. Zur Auswertung wird die Kalibrierung nach dem Ampullenbruch herangezogen.

| | |
|---|---|
| Wärmemenge: | 2,5Ws |
| Heizleistung: | 250 mW |
| Heizdauer: | 10 s |
| Dauer der Basislinien: | 5 min (vor und nach Heizen) |

Auswertung für Tiotropiumbromid-mikronisat:

Da die Masse des eingewogenen Tiotropiumbromid-mikronisats um den Wassergehalt des Materials korrigiert werden muss, werden die unverschlossenen Ampullen zusammen mit ca. 1 g der Testsubstanz mindestens 4 h offen stehen gelassen. Nach dieser Äquilibrierzeit werden die Ampullen mit den Silikonstopfen verschlossen und der Wassergehalt der Bulkprobe mittels Karl-Fischer-Titration bestimmt.

Die gefüllte und verschlossene Ampulle wird auf der Waage zurückgewogen. Die Korrektur der Probenmasse erfolgt nach folgender Formel:

$$m_c = \left(\frac{100\% - x}{100\%}\right) \cdot m_w$$

dabei sind:

$m_c$ korrigierte Masse
$m_w$ in die Ampulle eingewogene Probenmasse
x Wassergehalt in Prozent (mittels Karl-Fischer-Titration parallel bestimmt)

Die nach dieser Berechnung bestimmte korrigierte Masse $m_c$ wird als Eingabewert (= Einwaage) zur Berechnung der gemessenen Lösungsenthalphie verwendet.

**E) Darstellung der Pulverformullerung enthaltend das erfindungsgemäße Tiotropiumbromid-mikronisat**

In den nachfolgenden Beispielen wird als gröberer Hilfsstoff Lactose-Monohydrat (200M) verwendet. Dieser kann beispielsweise von der Firma DMV International, 5460 Veghel/NL unter der Produktbezeichnung Pharmatose 200M bezogen werden.

In den nachfolgenden Beispielen wird als feinerer Hilfsstoff Lactose-Monohydrat (5μ) verwendet. Dieser kann durch gängige Verfahren (Mikronisieren) aus Lactose-Monohydrat 200M erhalten werden. Lactose-Monohydrat 200M kann beispielsweise von der Firma DMV International, 5460 Veghel/NL unter der Produktbezeichnung Pharmatose 200M bezogen werden.

Apparatives

Zur Herstellung der das erfindungsgemäße Tiotropiumbromid-mikronisat enthaltenden Inhalationspulver können beispielsweise die folgenden Maschinen und Geräte Verwendung finden:

Mischbehälter bzw. Pulvermischer:

Rhönradmischer 200 L; Typ: DFW80N-4; Hersteller: Firma Engelsmann, D-67059 Ludwigshafen.

Siebgranulator:

Quadro Comil; Typ: 197-S; Hersteller: Firma Joisten & Kettenbaum, D-51429 Bergisch-Gladbach.

**E.1) Herstellung der Hilfsstoffmischung:**

Als gröbere Hilfsstoffkomponente werden 31,82 kg Lactose Monohydrat für Inhalationszwecke (200M) eingesetzt. Als feinere Hilfsstoffkomponente werden 1,68 kg Lactose Monohydrat (5μm) eingesetzt. In den daraus erhaltenen 33,5 kg Hilfsstoffmischung beträgt der Anteil der feineren Hilfsstoffkomponente 5%.

Über einen geeigneten Siebgranulator mit einem Sieb mit einer Maschenweite von 0,5 mm werden in einen geeigneten Mischbehälter ca. 0,8 bis 1,2 kg Lactose Monohydrat für Inhalationszwecke (200M) vorgelegt. Anschließend werden abwechselnd Lactose Monohydrat (5μm) in Portionen von ca. 0,05 bis 0,07 kg und Lactose Monohydrat für Inhalationszwecke (200M) in Portionen von 0,8 bis 1,2 kg schichtweise eingesiebt. Lactose Monohydrat für Inhalationszwecke (200M) und Lactose Monohydrat (5μm) werden in 31 bzw. in 30 Schichten (Toleranz: ±6 Schichten) zugegeben.

Die eingesiebten Bestandteile werden anschließend gemischt (Mischen: 900 Umdrehungen)

**E.2) Herstellung der Endmischung:**

Zur Herstellung der Endmischung werden 32,87 kg der Hilfsstoffmischung (1.1) und etwa 0,13 kg des erfindungsgemäßen Tiotropiumbromid-mikronisats eingesetzt. In den daraus erhaltenen 33,0 kg Inhalationspulver beträgt der Wirkstoffanteil 0.4%.

Über einen geeigneten Siebgranulator mit einem Sieb mit einer Maschenweite von 0,5 mm werden in einen geeigneten Mischbehälter ca. 1,1 bis 1,7 kg Hilfsstoffmischung (E.1) vorgelegt. Anschließend werden abwechselnd Tiotropiumbromid-mikronisat in Portionen von ca. 0,003 kg und Hilfsstoffmischung (E.1) in Portionen von 0,6 bis 0,8 kg schichtweise eingesiebt. Die Zugabe der Hilfststoffmischung und des Wirkstoffs erfolgt in 46 bzw. 45 Schichten (Toleranz: ± 9 Schichten).

Die eingesiebten Bestandteile werden anschließend gemischt (Mischen: 900 Umdrehungen). Die Endmischung wird noch zweimal über einen Siebgranulator gegeben und anschließend jeweils gemischt (Mischen: 900 Umdrehungen).

**E.3) Inhalationskapseln:**

Mit der nach E.2 erhaltenen Mischung werden Inhalationskapseln (Inhaletten) der folgenden Zusammensetzung erhalten:

| | |
|---|---|
| Tiotropiumbromid-mikronisat: | 0,0225 mg |
| Lactose Monohydrat (200 M): | 5,2025 mg |
| Lactose Monohydrat (5 μm): | 0,2750 mg |
| Hartqelatinekapsel: | 49,0 mq |
| Total: | 54,5 mg |

Unter analoger Anwendung der in E.2 beschriebenen Vorgehensweise werden ferner Inhalationskapseln (Inhaletten) der folgenden Zusammensetzung erhalten:

a)

| | |
|---|---|
| Tiotropiumbromid-mikronisat: | 0,0225 mg |
| Lactose Monohydrat (200 M): | 4,9275 mg |
| Lactose Monohydrat (5 μm): | 0,5500 mg |
| Hartqelatinekapsel: | 49,0 mq |
| Total: | 54,5 mg |

b)

| | |
|---|---|
| Tiotropiumbromid-mirkonisat: | 0,0225 mg |
| Lactose Monohydrat (200 M): | 5,2025 mg |
| Lactose Monohydrat (5 μm): | 0,2750 mg |
| Polyethylenkapsel: | 100,0 mq |
| Total: | 105,50 mg |

**F) Meßtechniken zur Partikelgrößenbestimmung der Hilfsstoffkomponenten, die in E) zur Anwendung gelangen**

Nachfolgend wird beschrieben, wie die Bestimmung der mittleren Teilchengröße der verschiedenen Hilfsstoffbestandteile der das erfindungsgemäße Tiotropiumbromidmikronisat enthaltenden und nach E) darstellbaren Formulierung erfolgen kann.

**F.1) Partikelgrößenbestimmung von feinteiliger Lactose:** Meßgerät und Einstellungen:

Die Bedienung der Geräte erfolgt in Übereinstimmung mit den Bedienungsanleitungen des Herstellers.

| | |
|---|---|
| Meßgerät: | HELOS Laser-Beugungs-Spektrometer, (SympaTec) |
| Dispergiereinheit: | RODOS Trockendispergierer mit Saugtrichter, (SympaTec) |
| Probenmenge: | ab 100 mg |
| Produktzufuhr: | Schwingrinne Vibri, Fa. Sympatec |
| Frequenz d. Vibrationsrinne: | 40 bis 100 % ansteigend |
| Dauer der Probenzufuhr: | 1 bis 15 sek. (im Fall von 100 mg) |
| Brennweite: | 100 mm (Meßbereich: 0,9 - 175 μm) |
| Meßzeit: | ca. 15 s (im Fall von 100 mg) |
| Zykluszeit: | 20 ms |
| Start/Stop bei: | 1 % auf Kanal 28 |
| Dispergiergas: | Druckluft |
| Druck: | 3 bar |
| Unterdruck: | maximal |
| Auswertemodus: | HRLD |

Probenvorbereitung / Produktzufuhr:

Mind. 100 mg der Prüfsubstanz werden auf einem Kartenblatt eingewogen. Mit einem weiteren Kartenblatt werden alle größeren Agglomerate zerstoßen. Das Pulver wird dann auf der vorderen Hälfte der Schwingrinne (ab ca. 1 cm vom vorderen Rand) fein verteilt aufgestreut. Nach dem Start der Messung wird die Frequenz der Schwingrinne von ca. 40 % bis 100 % (gegen Ende der Messung) variiert. Die Zeit, in der jeweils die gesamte Probe zugeführt wird beträgt 10 bis 15 sek.

**F.2) Partikelgrößenbestimmung von Laktose 200M :**

Meßgerät und Einstellungen

Die Bedienung der Geräte erfolgte in Übereinstimmung mit den Bedienungsanleitungen des Herstellers.

| | |
|---|---|
| Meßgerät: | Laser-Beugungs-Spektrometer (HELOS), Sympatec |
| Dispergiereinheit: | Trockendispergierer RODOS mit Saugtrichter, Sympatec |
| Probenmenge: | 500 mg |
| Produktzufuhr: | Vibrationsrinne Typ VIBRI , Sympatec |
| Frequenz d. Vibrationsrinne: | 18 bis 100 % ansteigend |
| Brennweite (1): | 200 mm (Meßbereich: 1.8 - 350 μm) |
| Brennweite (2): | 500 mm (Meßbereich: 4.5 - 875 μm) |
| Meßzeit/Wartezeit: | 10 s |
| Zykluszeit: | 10 ms |
| Start/Stop bei: | 1 % auf Kanal 19 |
| Druck: | 3 bar |
| Unterdruck: | maximal |
| Auswertemodus: | HRLD |

Probenvorbereitung / Produktzufuhr:

Ca. 500 mg der Prüfsubstanz werden auf einem Kartenblatt eingewogen. Mit einem weiteren Kartenblatt werden alle größeren Agglomerate zerstoßen. Das Pulver wird in der Trichter der Vibrationsrinne überführt. Es wird ein Abstand von 1.2 bis 1.4 mm zwischen Vibrationsrinne und Trichter eingestellt. Nach dem Start der Messung wird die Amplitudeneinstellung der Schwingrinne von 0 auf 40 % gesteigert bis sich ein kontinuierlicher Produktfluß einstellt. Danach wird auf eine Amplitude von ca. 18% reduziert. Gegen Ende der Messung wird die Amplitude auf 100% gesteigert.

## Patentansprüche

1. Kristallines Mikronisat von Tiotopiumbromid der Formel (I)

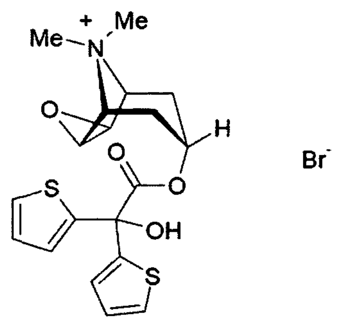

(I),

gekennzeichnet durch eine Partikelgröße $X_{50}$ von zwischen 1,0 μm und 3,5 μm bei einem Wert $Q_{(5.8)}$ von größer 60 %, **durch** einen spezifischen Oberflächenwert im Bereich zwischen 2 $m^2/g$ und 5 $m^2/g$, **durch** eine spezifische Lösungswärme von größer 65 Ws/g sowie **durch** einen Wassergehalt von 1 % bis 4,0 %.

2. Kristallines Tiotropiumbromid-Mikronisat nach Anspruch 1, **dadurch gekennzeichnet, dass** die Partikelgröße $X_{50}$ einen Wert von 1,1 μm bis 3,3 μm, bei einem Wert $Q_{(5.8)}$ von größer 70 % aufweist.

3. Kristallines Tiotropiumbromid-Mikronisat nach einem der Ansprüche 1 oder 2, **dadurch gekennzeichnet, dass** es einen spezifischen Oberflächenwert im Bereich von 2,5 $m^2/g$ bis 4,5 $m^2/g$ aufweist.

4. Kristallines Tiotropiumbromid-Mikronisat nach einem der Ansprüche 1, 2 oder 3, **gekennzeichnet durch** eine spezifische Lösungswärme von größer 71 Ws/g.

5. Kristallines Tiotropiumbromid-Mikronisat nach einem der Ansprüche 1 bis 4, **gekennzeichnet durch** einen Wassergehalt von 1,4 % bis 4,0 %.

6. Verwendung des kristallinen Tiotropiumbromid-Mikronisats nach einem der Ansprüche 1-5 zur Herstellung einer pharmazeutischen Zusammensetzung.

7. Verwendung von kristallinem Tiotropiumbromid-Mikronisat nach einem der Ansprüche 1-5 zur Herstellung eines Arzneimittels zur Behandlung von Erkrankungen, in denen die Applikation eines Anticholinergikums einen therapeutischen Nutzen entfalten kann.

8. Verwendung nach Anspruch 7, **dadurch gekennzeichnet, dass** es sich bei den Erkrankungen um Asthma oder COPD handelt.

9. Arzneimittel **gekennzeichnet durch** einen Gehalt an kristallinem Tiotropiumbromid-Mikronisat nach einem der Ansprüche 1-5.

10. Arzneimittel nach Anspruch 9, **dadurch gekennzeichnet, dass** es sich um ein Inhalationspulver handelt.

11. Inhalationspulver nach Anspruch 10, **dadurch gekennzeichnet, dass** es wenigstens 0,03 % Tiotropiumbromid-Mikronisat nach einem der Ansprüche 1-5 im Gemisch mit einem physiologisch unbedenklichen Hilfsstoff enthält, und ferner **dadurch gekennzeichnet, dass** der Hilfsstoff aus einem Gemisch von gröberem Hilfsstoff mit einer mittleren Teilchengröße von 15 bis 80 μm und feinerem Hilfsstoff mit einer mittleren Teilchengröße von 1 bis 9 μm besteht, wobei der Anteil von feinerem Hilfsstoff an der Gesamthilfsstoffmenge 1 bis 20 % beträgt.

12. Inhalationspulver nach Anspruch 11, **dadurch gekennzeichnet, dass** es zwischen 0,05 und 1 % Tiotropiumbromid-Mikronisat nach einem der Ansprüche 1-5 enthält.

13. Inhalationspulver nach einem der Ansprüche 11 oder 12, **dadurch gekennzeichnet, dass** der Hilfsstoff aus einem Gemisch von gröberem Hilfsstoff mit einer mittleren Teilchengröße von 17 bis 50 μm und feinerem Hilfsstoff mit einer mittleren Teilchengröße von 2 bis 8 μm besteht.

14. Inhalationspulver nach einem der Ansprüche 11, 12 oder 13, **dadurch gekennzeichnet, dass** der Anteil von feinerem Hilfsstoff an der Gesamthilfsstoffmenge 3 bis 15 % beträgt.

15. Inhalationspulver nach einem der Ansprüche 11-14, **dadurch gekennzeichnet, dass** als Hilfsstoffe Monosaccharide, Disaccharide, Oligo- und Polysaccharide, Polyalkohole, Salze oder Mischungen dieser Hilfsstoffe miteinander Verwendung finden.

16. Inhalationspulver nach Anspruch 15, **dadurch gekennzeichnet, dass** als Hilfsstoffe Glucose, Arabinose, Lactose, Saccharose, Maltose, Trehalose, Dextrane, Sorbit, Mannit, Xylit, Natriumchlorid, Calciumcarbonat oder Mischungen dieser Hilfsstoffe miteinander Verwendung finden.

17. Inhalationspulver nach Anspruch 16, **dadurch gekennzeichnet, dass** als Hilfsstoffe Glucose oder Lactose oder Mischungen dieser Hilfsstoffe miteinander Verwendung finden.

18. Verfahren zur Herstellung der Inhalationspulver nach einem der Ansprüche 11 bis 17, **dadurch gekennzeichnet, dass** in einem ersten Schritt die gröberen Hilfsstoffanteile mit den feineren Hilfsstoffanteilen gemischt werden und in einem anschließenden Schritt das so erhaltene Hilfsstoffgemisch mit dem Tiotropiumbromid-Mikronisat nach einem der Ansprüche 1-5 gemischt wird.

19. Kapsel (Inhalette), **gekennzeichnet durch** den Gehalt an einem Inhalationspulver gemäß einem der Ansprüche 10-17.

## Claims

1. Crystalline micronisate of tiotropium bromide of formula (I)

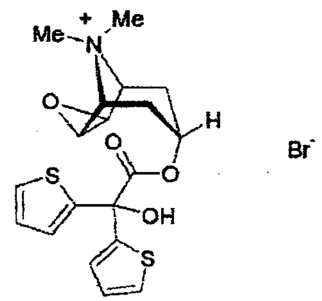

(I),

**characterised by** a particle size $X_{50}$ of between 1.0 μm and 3.5 μm at a $Q_{(5.8)}$ value of more than 60 %, by a specific surface value in the range between 2 $m^2/g$ and 5 $m^2/g$, by a specific heat of solution of more than 65 Ws/g and by a water content of 1 % to 4.0 %.

2. Crystalline tiotropium bromide micronisate according to claim 1, **characterised in that** the particle size $X_{50}$ has a value of 1.1 μm to 3.3 μm, at a $Q_{(5.8)}$ value of more than 70 %.

3. Crystalline tiotropium bromide micronisate according to one of claims 1 or 2, **characterised in that** it has a specific surface value in the range from 2.5 $m^2/g$ to 4.5 $m^2/g$.

4. Crystalline tiotropium bromide micronisate according to one of claims 1, 2 or 3, **characterised by** a specific heat of solution of more than 71 Ws/g.

5. Crystalline tiotropium bromide micronisate according to one of claims 1 to 4, **characterised by** a water content of 1.4 % to 4.0 %.

6. Use of the crystalline tiotropium bromide micronisate according to one of claims 1-5 for preparing a pharmaceutical composition.

7. Use of crystalline tiotropium bromide micronisate according to one of claims 1-5 for preparing a pharmaceutical composition for treating diseases in which the administration of an anticholinergic may have a therapeutic benefit.

8. Use according to claim 7, **characterised in that** the diseases are asthma or COPD.

9. Pharmaceutical composition **characterised in that** it contains crystalline tiotropium bromide micronisate according to one of claims 1-5.

10. Pharmaceutical composition according to claim 9, **characterised in that** it is an inhalable powder.

11. Inhalable powder according to claim 10, **characterised in that** it contains at least 0.03 % of tiotropium bromide micronisate according to one of claims 1-5 in admixture with a physiologically acceptable excipient, and further **characterised in that** the excipient consists of a mixture of coarser excipient with an average particle size of 15 to 80 μm and finer excipient with an average particle size of 1 to 9 μm, wherein the proportion of finer excipient in the total quantity of excipient is from 1 to 20%.

12. Inhalable powder according to claim 11, **characterised in that** it contains between 0.05 and 1 % of tiotropium bromide micronisate according to one of claims 1-5.

13. Inhalable powder according to one of claims 11 or 12, **characterised in that** the excipient consists of a mixture of coarser excipient with an average particle size of 17 to 50 μm and finer excipient with an average particle size of 2 to 8 μm.

14. Inhalable powder according to one of claims 11, 12 or 13, **characterised in that** the proportion of finer excipient in the total quantity of excipient is from 3 to 15 %.

15. Inhalable powder according to one of claims 11-14, **characterised in that** monosaccharides, disaccharides, oligo- and polysaccharides, polyalcohols, salts or mixtures of these excipients with one another are used as excipients.

16. Inhalable powder according to claim 15, **characterised in that** glucose, arabinose, lactose, saccharose, maltose, trehalose, dextrane, sorbitol, mannitol, xylitol, sodium chloride, calcium carbonate or mixtures of these excipients with one another are used as excipients.

17. Inhalable powder according to claim 16, **characterised in that** glucose or lactose or mixtures of these excipients with one another are used as excipients.

18. Process for preparing the inhalable powders according to one of claims 11 to 17, **characterised in that** in a first step the coarser excipient fractions are mixed with the finer excipient fractions and in a subsequent step the excipient mixture thus obtained is mixed with the tiotropium bromide micronisate according to one of claims 1-5.

19. Capsule (inhalette), **characterised in that** it contains an inhalable powder according to one of claims 10-17.

**Revendications**

1. Micronisat cristallin de bromure de tiotropium de la formule (I)

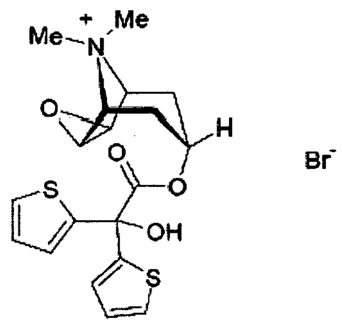

(I),

**caractérisé par** une taille de particule $X_{50}$ entre 1,0 μm et 3,5 μm pour une valeur $Q_{(5.8)}$ supérieure à 60 %, par une valeur de surface spécifique dans le domaine entre 2 $m^2$/g et 5 $m^2$/g, par une chaleur de dissolution spécifique supérieure à 65 Ws/g ainsi que par une teneur en eau de 1% à 4,0 %.

**2.** Micronisat cristallin de bromure de tiotropium selon la revendication 1, **caractérisé en ce que** la taille de particule $X_{50}$ présente une valeur de 1,1 μm à 3,3 μm, pour une valeur $Q_{(5.8)}$ supérieure à 70 %.

**3.** Micronisat cristallin de bromure de tiotropium selon l'une des revendications 1 ou 2, **caractérisé en ce qu'**il présente une valeur de surface spécifique dans la fourchette de 2,5 $m^2$/g à 4,5 $m^2$/g.

**4.** Micronisat cristallin de bromure de tiotropium selon l'une des revendications 1, 2 ou 3, **caractérisé par** une chaleur de dissolution spécifique supérieure à 71 Ws/g.

**5.** Micronisat cristallin de bromure de tiotropium selon l'une des revendications 1 à 4, **caractérisé par** une teneur en eau de 1,4 % à 4,0 %.

**6.** Utilisation du micronisat cristallin de bromure de tiotropium selon l'une des revendications 1-5 pour la production d'une composition pharmaceutique.

**7.** Utilisation du micronisat cristallin de bromure de tiotropium selon l'une des revendications 1- 5 pour la production d'un médicament pour le traitement des maladies dans lesquelles l'application d'un anticholinergique peut se révéler être d'une utilité thérapeutique.

**8.** Utilisation selon la revendication 7, **caractérisée en ce que** les maladies sont l'asthme ou la COPD.

**9.** Médicament **caractérisé par** une teneur en micronisat cristallin de bromure de tiotropium selon l'une quelconque des revendications 1-5.

**10.** Médicament selon la revendication 9, **caractérisé en ce qu'**il s'agit d'une poudre pour inhalation.

**11.** Poudre pour inhalation selon la revendication 10, **caractérisée en ce qu'**elle contient au moins 0,03 % de micronisat de bromure de tiotropium selon l'une des revendications 1 - 5 en mélange avec un adjuvant physiologiquement acceptable et **caractérisée en outre en ce que** l'adjuvant consiste en un mélange d'adjuvant plus grossier ayant une taille de particule moyenne de 15 à 80 μm et d'adjuvant plus fin ayant une taille de particule moyenne de 1 à 9 μm, où la fraction d'adjuvant plus fin par rapport à la quantité totale d'adjuvants est de 1 à 20 %.

**12.** Poudre pour inhalation selon la revendication 11, **caractérisée en ce qu'**elle contient entre 0,05 et 1 % de micronisat de bromure de tiotropium selon l'une des revendications 1-5.

**13.** Poudre pour inhalation selon l'une des revendications 11 ou 12, **caractérisée en ce que** l'adjuvant consiste en un mélange d'adjuvant plus grossier ayant une taille de particule moyenne de 17 à 50 μm et d'adjuvant plus fin ayant une taille de particule moyenne de 2 à 8 μm.

**14.** Poudre pour inhalation selon l'une des revendications 11, 12 ou 13, **caractérisée en ce que** la fraction de l'adjuvant plus fin par rapport à la quantité totale d'adjuvants est 3 à 15 %.

**15.** Poudre pour inhalation selon l'une des revendications 11- 14, **caractérisée en ce que** des monosaccharides, des disaccharides, des oligo- et polysaccharides, des polyalcools, des sels ou des mélanges de ces adjuvants entre eux sont utilisés comme adjuvants.

**16.** Poudre pour inhalation selon la revendication 15, **caractérisée en ce que** le glucose, l'arabinose, le lactose, le saccharose, le maltose, le tréhalose, les dextranes, le sorbitol, le mannitol, le xylitol, le chlorure de sodium, le carbonate de calcium ou des mélanges de ces adjuvants entre eux sont utilisés comme adjuvants.

**17.** Poudre pour inhalation selon la revendication 16, **caractérisée en ce que** le glucose ou le lactose ou des mélanges de ces adjuvants entre eux sont utilisés comme adjuvants.

**18.** Procédé de production des poudres pour inhalation selon l'une des revendications 11 à 17, **caractérisé en ce que**, dans une première étape, les fractions d'adjuvant plus grossières sont mélangées avec les fractions d'adjuvant plus fines et, dans une étape subséquente, le mélange d'adjuvants ainsi obtenu est mélangé avec le micronisat de bromure de tiotropium selon l'une des revendications 1-5.

**19.** Capsule (inhalette) **caractérisée par** la teneur en une poudre pour inhalation selon l'une des revendications 10 - 17.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 418716 A1
- WO 0047200 A
- FR 2779347
- US 4570630 A
- DE 3625685 A
- WO 9428958 A